(19) Europäisches Patentamt
European Patent Office
Office européen des brevets

(11) Veröffentlichungsnummer: **0 137 417**
**B1**

(12) # EUROPÄISCHE PATENTSCHRIFT

(45) Veröffentlichungstag der Patentschrift: **05.11.86**

(51) Int. Cl.⁴: **C 07 C 147/12**, C 07 C 147/14

(21) Anmeldenummer: **84111510.8**

(22) Anmeldetag: **27.09.84**

(54) **Verfahren zur Herstellung von Aminobenzol-alkylsulfonen bzw. -sulfoxiden.**

(30) Priorität: **08.10.83 DE 3336751**

(43) Veröffentlichungstag der Anmeldung: **17.04.85 Patentblatt 85/16**

(45) Bekanntmachung des Hinweises auf die Patenterteilung: **05.11.86 Patentblatt 86/45**

(84) Benannte Vertragsstaaten: **CH DE FR GB LI**

(56) Entgegenhaltungen:
**EP-A-0 007 880**
**EP-A-0 035 956**

**THE JOURNAL OF ORGANIC CHEMISTRY, Band 47, 1982, CLIFFORD G. VENIER et al. "Peroxytrifluoroacetic Acid. A convenient reagent for the preparation of sulfoxides and sulfons"**
**CHEMICAL ABSTRACTS, Band 51, Nr. 2, 25. Januar 1957, Columbus, Ohio, USA, O. ACHMATOWICZ et al. "Sulfonic acids. I. Addition of sulfonic acid salts to alpha, beta-unsaturated compounds. Synthesis of sulfones", Spalte 1064b**

(73) Patentinhaber: **BAYER AG, Konzernverwaltung RP Patentabteilung, D-5090 Leverkusen 1 Bayerwerk (DE)**

(72) Erfinder: **Michna, Martin, Dr., Silesiusstrasse 74, D-5000 Köln (DE)**
Erfinder: **Henk, Hermann, Dr., Roggendorferstrasse 55, D-5000 Köln 80 (DE)**
Erfinder: **Herd, Karl Josef, Dr., Buchholzstrasse 32, D-5000 Köln 80 (DE)**

EP 0 137 417 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung ist ein Verfahren zur Herstellung von Sulfonen bzw. Sulfoxiden der Formel

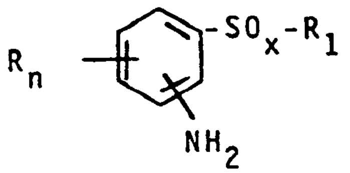

(I)

worin

R = H oder gegebenenfalls substituiertes $C_1$-$C_4$-Alkyl, gegebenenfalls substituiertes Phenyl, Nitro, Sulfo, CN, Halogen, OH, SH, $NH_2$, gegebenenfalls substituiertes $C_1$-$C_4$-Alkoxy, gegebenenfalls substituiertes $C_1$-$C_4$-Alkoxy, gegebenenfalls substituiertes Phenylazo, gegebenenfalls weiter substituiertes Benz-triazolyl-2- oder Naphthriazolyl-2,

$R_1$ = gegebenenfalls durch Chlor, gegebenenfalls substituiertes Phenyl, Nitro, Sulfo, CN, OH, SH, $NH_2$, Aminocarbonyl, gegebenenfalls substituiertes $C_1$-$C_4$-Alkoxy, den Rest

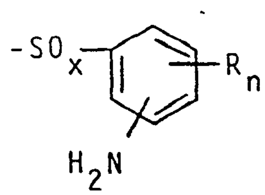

substituiertes $C_1$-$C_4$-Alkyl,
n = 1 bis 4
x = 1 oder 2
durch Oxidation von Sulfiden der Formel

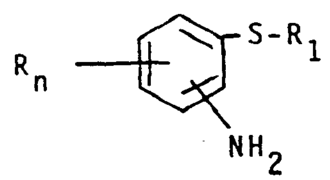

(II)

dadurch gekennzeichnet, daß man die Oxidation in wäßrigem Medium bei pH-Werten von 3 bis 14 durchführt.

Vorzugsweise wird bei PH-Werten von 5 bis 14, insbesondere bei PH-Werten von 7 bis 14 oxidiert.

Die Oxidation kann in Lösung, Dispersion oder Emulsion erfolgen. Neben Wasser können auch unter den Reaktionsbedingungen stabile organische wassermischbare Lösungsgittel verwendet werden. Vorzugsweise beträgt der Wassergehalt des Reaktionsgemisches dabei mindestens 50 %. Im allgemeinen wird bei Temperaturen von etwa 0 bis 120° C, vorzugsweise 0 bis 60° C gearbeitet.

Geeignete Oxidationsmittel sind beispielsweise: Sauerstoff, Halogene wie Chlor oder Brom, Permanganate, Chromate, Nitrate, Eisen-III-salze und insbesondere Peroxide wie Wasserstoffperoxid sowie Perverbindungen wie Perborate, Persulfate, Percarbonate und Persulfonsäuren. Gegebenenfalls können geeignete Katalysatoren zugesetzt werden, beispielsweise Wolframate, Vanadate, Molybdate und Eisen-III-salze.

Die Oxidationsmittel werden im allgemeinen in Mengen von etwa 1 bis 8 Mol pro Mol II eingesetzt.

Die Substituenten R können gleich oder verschieden sein. Geeignete Substituenten sind beispielsweise gegebenenfalls substituiertes $C_1$-$C_4$-Alkyl, gegebenenfalls substituiertes Phenyl, Nitro, Sulfo, CN, Halogen, insbesondere Cl, OH, SH, $NH_2$, gegebenenfalls substituiertes $C_1$-$C_4$-Alkoxy, gegebenenfalls substituiertes Phenylazo, gegebenenfalls weiter substituiertes Benztriazolyl-2 oder Naphthtriazolyl-2.

Geeignete Reste $R_1$ sind gegebenenfalls durch Chlor, gegebenenfalls substituiertes Phenyl, Nitro, Sulfo, CN, OH, SH, $NH_2$, Aminocarbonyl, gegebenenfalls substituiertes $C_1$-$C_4$-Alkoxy oder den Rest

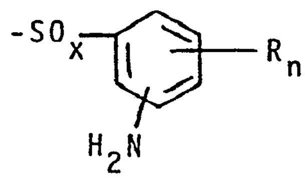

substituiertes $C_1$-$C_4$-Alkyl.

Die Verbindungen I sind bekannt. Sie werden beispielsweise als Diazokomponenten für die Herstellung von Azofarbstoffen eingesetzt und wurden bislang beispielsweise durch Oxidation der Verbindungen II in wasserfreien Säuren wie Eisessig hergestellt (J. Org. Chem. 1982, 47, Seite 3773 bis 3774; Farm. Ed. Sci. 35(4), 1980, S. 279 bis 297). Völlig überraschend wurde nun gefunden, daß die Oxidation auch in wäßrigem Medium durchgeführt werden kann, ohne dass die freie Aminogruppe angegriffen wird.

**Beispiele**

**Beispiel 1**

(2-Hydroxyethyl)-(2-aminophenyl)-sulfon

5 Mol (2-Hydroxyethyl)-(2-aminophenyl)-sulfid werden in 4 l Wasser emulgiert und mit 8 g Natriumwolframat versetzt. Der PH-Wert wird auf 7 eingestellt. Danach werden 860 ml 35 %iges Wasserstoffperoxid im Verlauf von 2 Stunden zugetropft. Die Reaktion ist stark exotherm. Die Temperatur wird durch Kühlung bei etwa 50° C gehalten. Man rührt nach bis kein $H_2O_2$ mehr nachweisbar ist und läßt abkühlen. Das ölige Reaktionsprodukt wird abgetrennt. Ausbeute: 85 % der Theorie.

**Beispiel 2**

(2-Hydroxyethyl)-(2-aminophenyl)-sulfoxid

Der Ansatz wird wie im Beispiel 1 durchgeführt. Es wird jedoch nur die halbe Menge Wasserstoffperoxid in 2 Stunden zugetropft. Die Temperatur wird bei 30 bis 40° C gehalten. Ausbeute: 90 % der Theorie.

**Beispiel 3**

(2-Sulfoethyl)-(2-aminophenyl)-sulfon

0,66 Mol (2-Sulfoethyl)-(2-aminophenyl)-sulfid werden mit 600 ml Wasser verrührt. Man stellt den pH-Wert auf 9 und gibt 2,3 g Natriumwolframat zu. Nun tropft man langsam 115 ml (1,32 Mol) 35 %iges Wasserstoffperoxid zu, wobei die Temperatur bis auf 50°C steigt. Man läßt nachrühren, bis kein $H_2O_2$ mehr nachweisbar ist und kühlt auf Raumtemperatur ab. Das Reaktionsprodukt wird durch Zusatz von 12,5 % NaCl und 12,5 % KCl ausgesalzen, abfiltriert und getrocknet. Ausbeute: 87 % der Theorie.

**Beispiel- 4**

(2-Sulfoethyl)-(2-aminophenyl)-sulfoxid

Der Ansatz wird wie in Beispiel 3 durchgeführt. Es wird jedoch die halbe Menge Wasserstoffperoxid in 2 Stunden zugetropft. Die Temperatur wird zwischen 30° C und 40° C gehalten. Ausbeute: 80 % der Theorie.

**Beispiel 5**

(4-Nitrobenzyl)-(2-aminophenyl)-sulfon

0,5 Mol (4-Nitrobenzyl)-(2-aminophenyl)-sulfid werden unter Zusatz eines üblichen oberflächenaktiven Mittels in 1500 ml Wasser gut verrührt. Man stellt den pH-Wert auf 4 bis 4,5 ein, gibt 2 g Natriumwolframat zu und erhitzt zum Sieden. In die siedende Mischung tropft man in ca. 2 Stunden 110 ml 35 %iges

Wasserstoffperoxid. Anschließend wird noch 2 Stunden am Rückfluß gekocht. Danach läßt man auf Raumtemperatur abkühlen. Das zunächst ölige Reaktionsprodukt kristallisiert durch und kann abgesaugt werden. Ausbeute: 62 % der Theorie.

**Beispiel 6**

3-(2-Aminophenyl)-sulfonylpropionsäureamid

0,1 Mol 3-(2-Aminophenyl)thiopropionsäureamid werden in Wasser suspendiert, der pH-Wert auf 10 gestellt und auf 60°C aufgeheizt. Man gibt 2 g Natriumwolframat zu und tropft anschließend im Verlauf von ca. 2 Stunden 19 ml Wasserstoffperoxidlösung (35 %ig) zu. Die Reaktion ist stark exotherm. Man rührt bei 60°C bis kein $H_2O_2$ mehr nachweisbar ist, kühlt ab und extrahiert die Reaktionsmischung mit Methylenchlorid. Die organische phase wird abgetrennt, getrocknet und das Lösungsmittel im Vakuum entfernt. Das zurückbleibende Öl kristallisiert zu einer hellgelben Masse. Ausbeute: 61 % der Theorie.

**Beispiel 7**

(2-Aminoethyl)-(2-aminophenyl)-sulfon

1 Mol (2-Aminoethyl)-(2-aminophenyl)-sulfid werden unter Zusatz eines üblichen Emulgators in 800 ml Wasser emulgiert. Man stellt mit Eisessig pH 5 ein, kühlt auf 0°C, setzt 5 g Natriumwolframat zu und tropft zu dieser Mischung langsam bei 0°C 205 ml Wasserstoffperoxidlösung (35 %ig). Man rührt nach bis kein $H_2O_2$ mehr nachweisbar ist, trennt das ölige Reaktionsprodukt ab und destilliert im Hochvakuum. Ausbeute (roh) 65 % der Theorie.

**Beispiel 8**

(2-Hydroxyethyl)-(4-aminophenyl)-sulfon

1 Mol (2-Hydroxyethyl)-(4-aminophenyl)-sulfid werden in 800 ml Wasser verrührt und mit 1,5 g Natriumwolframat versetzt. Man stellt den pH-Wert auf 8 und tropft in 2 Stunden 190 ml Wasserstoffperoxidlösung (35 %ig) zu. Das ölige Reaktionsprodukt wird von der wäßrigen phase abgetrennt und im Vakuum getrocknet. Ausbeute: 92 % der Theorie.

**Beispiel 9**

Methyl-(2-aminophenyl)-sulfoxid

1 Nol Methyl-(2-aminophenyl)-sulfid werden in 800 ml Wasser emulgiert. Man stellt den pH-Wert auf 8, setzt 2 g Natriumwolframat zu und tropft in ca. 2 Stunden 1 bis 1,2 Mol 35 %iges Wasserstoffperoxid zu. Die Temperatur wird durch Kühlung bei 30°C gehalten. Man rührt nach, bis kein Wasserstoffperoxid mehr nachweisbar ist. Das ölige Reaktionsprodukt wird wie üblich abgetrennt und getrocknet. Ausbeute: 85 % der Theorie.

**Patentansprüche**

1. Verfahren zur Herstellung von Sulfonen bzw. Sulfoxiden der Formel

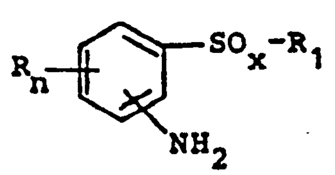

(I)

worin

R = H oder gegebenenfalls substituiertes $C_1$-$C_4$-Alkyl, gegebenenfalls substituiertes Phenyl, Nitro, Sulfo,

CN, Halogen, OH, SH, $NH_2$, gegebenenfalls substituiertes $C_1$-$C_4$-Alkoxy, gegebenenfalls substituiertes Phenylazo, gegebenenfalls weiter substituiertes Benztriazolyl-2- oder Naphthtriazolyl-2,

$R_1$ = gegebenenfalls durch Chlor, gegebenenfalls substituiertes Phenyl, Nitro, sulfo, CN,OH, SH, $NH_2$, Aninocarbonyl, gegebenenfalls substituiertes $C_1$-$C_4$-Alkoxy, den Rest

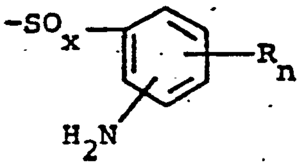

(II)

substituiertes $C_1$-$C_4$-Alkyl,

n = 1 bis 4

x = 1 oder 2

durch Oxidation von Sulfiden der Formel

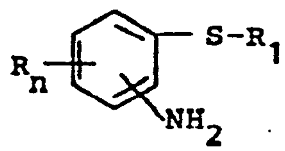

dadurch gekennzeichnet, daß man die Oxidation in wäßrigen Medium bei pH-Werten von 3 bis 14 durchführt.

2. Verfahren nach Anspruch 1, dadurch gekennzeichnet, daß man bei pH-Werten von 5 bis 14 oxidiert.

3. Verfahren nach Ansprüchen 1 und 2, dadurch gekennzeichnet, daß man mit dem System Wasserstoffperoxid - Wolfram oxidiert.

## Claims

1. Process for the preparation of sulphones and sulphoxides of the formula

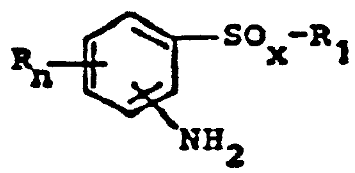

(I)

wherein

R = H or optionally substituted $C_1$-$C_4$-alkyl, optionally substituted phenyl, nitro, sulpho, CN, halogen, OH, SH, $NH_2$, optionally substituted $C_1$-$C_4$-alkoxy, optionally substituted phenylazo, optionally further substituted benzotriazol-2-yl or naphthotriazol-2-yl,

$R_1$ = $C_1$-$C_4$-alkyl which is optionally substituted by chlorine, optionally substituted phenyl, nitro, sulpho, CN, OH, SH, $NH_2$, aminocarbonyl, optionally substituted $C_1$-$C_4$-alkoxy or the radical

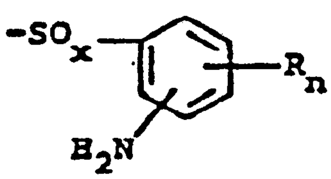

(II)

n = 1 to 4,

x = 1 or 2,

by the oxidation of sulphides of the formula

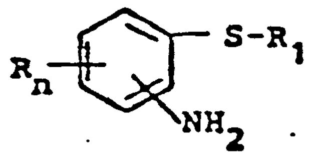

characterised in that the oxidation is carried out in an aqueous medium at pH values of 3 to 14.

2. Process according to Claim 1, characterised in that the oxidation is carried out at pH values of 5 to 14.

3. Process according to Claims 1 and 2, characterised in that the oxidation is carried out using the hydrogen peroxide/tungsten system.

**Revendications**

1. Procédé pour la fabrication de sulfones ou sulfoxydes de formule

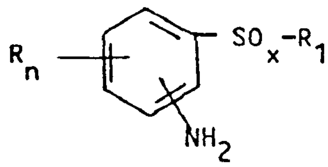

(I)

dans laquelle

R est un atome d'hydrogène ou d'halogène ou un groupe alkyle en $C_1$-$C_4$ éventuellement substitué, phényle éventuellement substitué, nitro, sulfo, CN, OH, SH, $NH_2$, alcoxy en $C_1$-$C_4$ éventuellement substitué, phénylazo éventuellement substitué, benzotriazolyle-2 ou naphto-triazolyle-2 encore éventuellement substitué,

$R_1$ est un groupe alkyle en $C_1$-$C_4$ éventuellement substitué par le chlore, par un groupe phényle éventuellement substitué, par un groupe nitro, sulfo, CN, OH, SH, $NH_2$ ou aminocarbonyle, par un groupe alcoxy en $C_1$-$C_4$ éventuellement substitué, ou par le reste

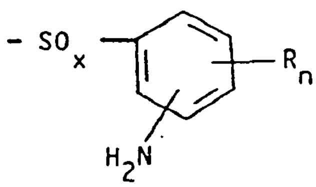

n = 1 à 4

x = 1 ou 2

par oxydation de sulfures de formule

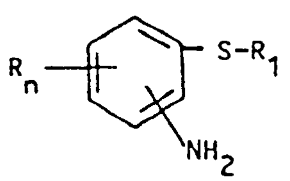

(II)

caractérisé en ce qu'on met en oeuvre l'oxydation en milieu aqueux à des pH de 3 à 14.

2. Procédé selon la revendication 1, caractérisé en ce que l'on oxyde à des pH de 5 à 14.

3. Procédé selon les revendications 1 et 2, caractérisé en ce que l'on oxyde avec le système peroxyde d'hydrogène-tungstène.